## Europäisches Patentamt
## European Patent Office
## Office européen des brevets

(11) Veröffentlichungsnummer: **0 153 546**
**B1**

# EUROPÄISCHE PATENTSCHRIFT

(45) Veröffentlichungstag der Patentschrift:
**27.01.88**

(51) Int. Cl.⁴: **A 61 B 17/60**

(21) Anmeldenummer: **84810116.8**

(22) Anmeldetag: **08.03.84**

(54) **Fixateur zum Fixieren von Knochenstücken.**

(30) Priorität: **19.01.84 CH 230/84**

(43) Veröffentlichungstag der Anmeldung:
**04.09.85 Patentblatt 85/36**

(45) Bekanntmachung des Hinweises auf die Patenterteilung:
**27.01.88 Patentblatt 88/4**

(84) Benannte Vertragsstaaten:
**AT DE FR GB SE**

(56) Entgegenhaltungen:
**FR - A - 2 439 002**
**GB - A - 2 110 094**
**US - A - 2 393 694**
**US - A - 4 312 336**

(73) Patentinhaber: **SYNTHES AG, Grabenstrasse 15, CH-7002 Chur (CH)**

(72) Erfinder: **Comte, Pierre-André, Dr.,**
**Seltisbergerstrasse 19, CH-4410 Liestal (CH)**
Erfinder: **Burri, Caius, Prof. Dr. med.,**
**Steinhövelstrasse 9, D-7900 Ulm (DE)**
Erfinder: **Claes, Lutz, Dr., Oberer Eselberg, D-7900 Ulm (DE)**
Erfinder: **Gerngross, Heinz, Dr. med.,**
**Steinhövelstrasse 9, D-7900 Ulm (DE)**
Erfinder: **Meier, Remi, Mittebrühlstrasse 1, CH-4416 Bubendorf (CH)**

(74) Vertreter: **Eder, Carl E., Patentanwaltsbüro EDER AG**
**Münchensteinerstrasse 2, CH-4052 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft einen Fixateur gemäss dem Oberbegriff des Anspruches 1.

Ein derartiger Fixateur kann verwendet werden, um nach einer Fraktur oder Osteotomie eines Knochens, insbesondere des in einem Beim oder Arm eines Patienten vorhandenen Knochens, die Knochenstücke oder -fragmente mit Stiften zu fixieren, die von der Aussenseite des Körpers her durch die Haut hindurch in die Knochenstücke eingesetzt, vorzugsweise eingeschraubt werden. Der Fixateur befindet sich also bei seiner Verwendung abgesehen von den einen Endabschnitten seiner Stifte, ausserhalb des Körpers des Patienten. Bei der Behandlung einer Knochenfraktur werden die Knochenstücke üblicherweise zuerst provisorisch mit dem Fixateur fixiert und dann durch Verstellen von Teilen des letzteren nachträglich justiert. Eventuell können die Knochenstücke bereits an einem Unfallort für den Transport provisorisch mit dem Fixateur fixiert und dann im Spital justiert werden.

Ein aus der Schweizerpatentschrift 608 712 bekannter Fixateur besitzt den zu fixierenden Knochen durchdringende Nägel, deren Enden an auf verschiedenen Seiten des Knochens angeordneten Streben befestigt sind. Jede Strebe ist aus zwei Stangen gebildet, die durch ein Gelenk miteinander verbunden sind. Jedes Gelenk weist zwei Gelenkteile auf, in denen je eine der Stangen um ihre Längsachse verschwenkbar und in verschiedenen Schwenkstellungen festklemmbar gehalten ist. Die beiden Gelenkteile sind ihrerseits um einen sie verbindenden Gelenkbolzen schwenkbar und in verschiedenen Schwenkstellungen fixierbar. Zur Befestigung der Nägel an den Streben sind Klemmvorrichtungen mit einem an den Streben festklemmbaren Teil und einem bezüglich diesem schwenkbaren, die Nägel verschiebbar festklemmenden Teil vorhanden.

Bei diesen aus der Schweizerpatentschrift 608 712 bekannten Fixateur muss also auf beiden Seiten des Knochens eine Strebe angeordnet werden, wodurch der Patient beträchtlich behindert wird. Ferner ist es mit diesem Fixateur trotz der grossen Anzahl verstellbarer Teile praktisch nicht möglich, die Knochenbruchstücke nach dem Einsetzen der Nägel zum Jusieren ihrer Lage gegeneinander um die Längsachse des Knochens zu verdrehen. Dies wirkt sich insbesondere in Fällen stark nachteilig aus, bei denen die Knochenstücke direkt am Unfallort für den Abtransport des Verletzten mit einem Fixateur provisorisch fixiert werden sollten, weil es dann am Unfallort meistens nicht möglich ist, die Knochenstücke bereits derart zu richten, dass sie nachher durch Verstellen von Fixateurteilen in die richtige Lage gebracht werden können. Ferner wäre es in vielen Fällen wünschenswert, die Knochenstücke nach der provisorischen Fixierung zu justieren und bei der Frakturstelle gegeneinander zu drücken, was mit dem bekannten Fixateur ebenfalls nicht oder höchstens unter Verwendung zusätzlicher Spannmittel möglich ist. Im übrigen hat sich gezeigt, dass der Fixateur in der Praxis häufig nicht ausreichend stabil war, so dass man ihn wenn möglich ohne die Gelenke benutzte.

Aus der US-Patentschrift 4 312 336 ist ferner ein Fixateur gemäss Oberbegriff des Anspruchs 1 mit zwei Haltern bekannt, in denen zum Einschrauben in Knochenstücke bestimmte Stifte entlang ihrer Achse verstellbar festgeklemmt sind. Ein Verbindungsglied weist eine Hülse und einen axial verschiebbar in dieser geführten sowie in verschiedenen Schiebstellungen fixierbaren Dorn auf. Die Hülse und der Dorn sind je durch ein Kugelgelenk mit einem der beiden Halter verbunden. Jedes Kugelgelenk weist dabei einen mehrteiligen Gelenkkörper auf, in dem eine Gelenkkugel gelagert ist. Zum Festklemmen der letzteren ist der Gelenkkörper mit einem festschraubbaren Gewindering sowie mit zwei Klemmschrauben versehen. Eine Spanneinrichtung zur Erzeugung einer axialen Spannkraft weist eine Gewindebuchse und eine Lagerbuchse zum Lagern einer in die Gewindebuchse eingeschrauben Spannschraube auf. Die beiden Buchsen sind je mit einem quer zu ihren Achsen verlaufenden Befestigungsbolzen versehen, die in Querbohrungen der Hülse und des Dorns des Verbindungsgliedes steckbar sind.

Bei diesem Fixateur können zwar die beiden Halter als Ganzes bezüglich einander um die Längsachse des Verbindungsgliedes gedreht werden. Die vom gleichen Halter gehaltenen Stifte müssen jedoch zwangsläufig alle parallel zueinander verlaufen, wodurch die Möglichkeiten zum Justieren der Knochenstücke stark beschränkt werden. Da die ja mehrere Stifte haltenden Halter bezüglich der Kugelgelenke verhältnismässig lange Hebelarme bilden, können sie bei der Benutzung des Fixateurs grosse Drehmomente auf die Kugelgelenke ausüben, so dass trotz der aufwendigen Konstruktion dieser Kugelgelenke und der zu ihrer Fixierung dienenden Fixiermittel die Gefahr besteht, dass sich die Kugelgelenke bei Belastungen auch im fixierten Zustand bewegen. Des weitern erschwert es die Handhabung und Benutzung des Fixateurs, dass die Spanneinrichtung nicht dauernd in den Fixateur integriert, sondern bei der Benutzung zuerst an dessen Verbindungsglied angebracht werden muss und dann neben dem Verbindungsglied entlang von diesem verläuft. Zudem ist die Herstellung des Fixateurs sehr aufwendig.

Der Vollständigkeit halber sei auch noch der aus der Schweizerpatentschrift 633 174 bekannte Fixateur erwähnt, der jedoch ausschliesslich für die Fixierung vovn Wirbelknochen vorgesehen ist.

Die Erfindung hat sich nun zur Aufgabe gestellt, den aus der US-Patentschrift 4 312 336 bekannten Fixateur dahingehend zu verbessern, dass zwischen den beiden Haltern und dem diese miteinander verbindenden Verbindungsglied keine im fixierten Zustand die Stabilität beeinträchtigenden Kugelgelenke mehr notwendig sind und trotzdem eine ausreichende Verstellbarkeit der gehaltenen Knochenstücke erzielt werden kann.

Diese Aufgabe wird durch einen Fixateur gelöst, der nach der Erfindung gemäss dem Anspruch 1 ausgebildet ist. Vorteilhafte Weiterbildungen des Fixateurs gehen aus den abhängigen Ansprüchen hervor.

Der Erfindungsgegenstand soll nun anhand eines in der Zeichnung dargestellten Ausführungsbeispiels erläutert werden.

In der Zeichnung zeigen:

die Fig. 1 eine Seitenansicht eines Knochens und eines Fixateurs,

die Fig. 2 einen Längsschnitt durch den in der Fig. 1 dargestellten Fixateur, wobei aber einige der einschraubbaren Stifte weggelassen wurden,

die Fig. 3 einen Schnitt entlang der Linie III-III der Fig. 1, in grösserem Massstab,

die Fig. 4 einen Schnitt entlang der Linie IV-IV der Fig. 1,

die Fig. 5 einen Schnitt entlang der Linie V-V der Fig. 1, wobei einige Teile weggelassen wurden,

die Fig. 6 einen Schnitt entlang der Linie VI-VI der Fig. 1,

die Fig. 7 einen Schnitt entlang der Linie VII-VII der Fig. 1,

die Fig. 8 eine Ansicht von einem Abschnitt des Dorns in der in der Fig. 7 durch den Pfeil VIII bezeichneten Blickrichtung und

die Fig. 9 eine Draufsicht auf die vom Fortsatz des Dorns gebildete Auflagefläche.

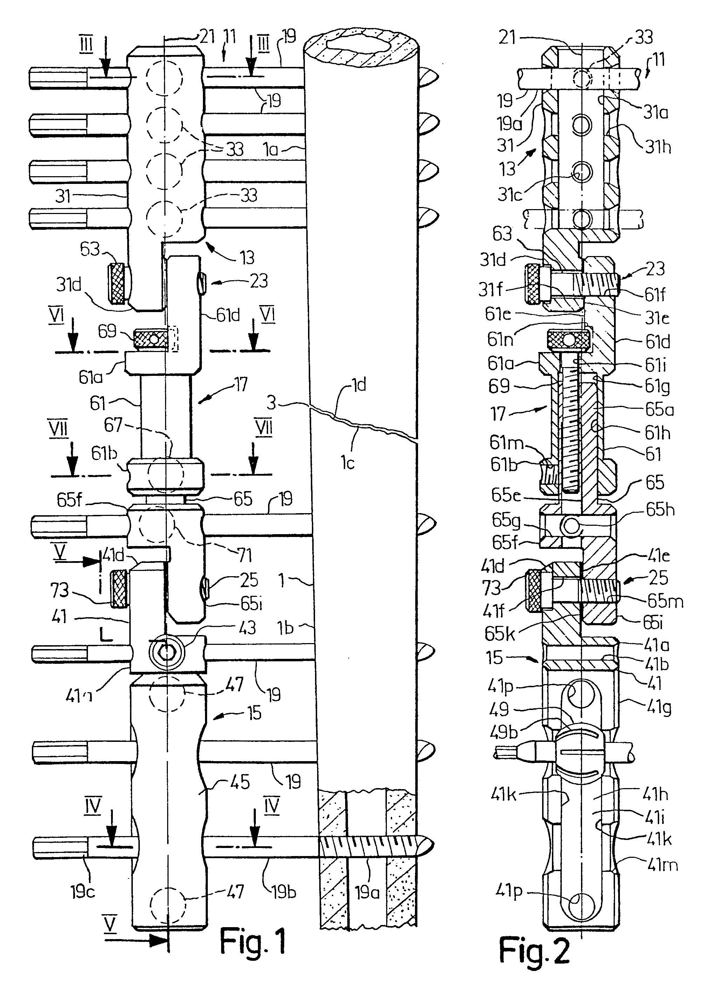

In der Fig. 1 ist ein Abschnitt eines menschlichen Knochens 1, nämlich eines Röhrenknochens eines Beins oder eventuell Arms ersichtlich, der bei einer Frakturstelle 3 in zwei Knochenstücke oder -fragmente 1a, 1b gebrochen ist, wobei die Frakturfläche der beiden Knochenstücke oder -fragmente mit 1c bzw. 1d bezeichnet sind. Die Knochenstücke 1a, 1b sind mit einem externen Fixateur 11 bezüglich einander fixiert. Der Fixateur weist zwei Halter 13 und 15 auf, die durch ein zwischen ihnen angeordnetes Verbindungsglied 17 miteinander verbunden sind. Ferner sind in den Knochen 1 eingeschraubte Stifte 19, sogenannte Schanz'sche Schrauben vorhanden, von denen jede einen Gelenkteil 19a, einen zylindrischen Schaft 19b und einen sechskantförmigen Ansatz 19c zum Angreifen mit einem Schlüssel aufweist. Die beiden Halter 13, 15 und das Verbindungsglied 17 sind ausserhalb des Körpers des Patienten angeordnet und die Schäfte der Stifte 19 dringen durch die nicht dargestellte Haut des Patienten in dessen Körper ein. Beim dargestellten Fixateur kann der Halter 13 vier Stife, das Verbindungsglied 17 einen Stift und der Halter 15 drei Stifte lösbar halten. Das Verbindungsglied 17 ist durch ein Gelenk 23 mit dem Halter 13 und durch ein Gelenk 25 mit dem Halter 15 verbunden.

Die beiden Halter 13, 15 und das Verbindungsglied 17 sind alle länglich ausgebildet und können bezüglich einander in Stellungen fixiert werden, in denen sie entlang einer gemeinsamen Längsachse 21 verlaufen. Im übrigen sind die Umfangs-Hüllflächen der Halter und auch des Verbindungsgliedes abgesehen von den vorstehenden Schrauben im allgemeinen mindestens ungefähr kreiszylindrisch sowie in der genannten Stellung koaxial zur Längsachse 21 und haben im übrigen auch alle ungefähr den gleichen Durchmesser.

Die Ausbildung des Halters 13 ist aus den Fig. 1, 2 und 3 ersehbar. Der Hauptteil des Halters besteht aus einem im allgemeinen kreiszylindrischen, einstückigen Träger 31 mit einem zu seiner Längsmittelachse parallelen, aber exzentrischen, in sein dem Verbindungsglied 17 abgewandtes Ende mündenden, zur Material- und Gewichtsersparnis dienenden Längsloch 31a, nämlich einer Sackbohrung. Der Träger 31 ist für jeden von ihm zu haltenden Stift 19 mit einem seine Längsachse und das Längsloch 31a rechtwinklig kreuzenden Durchgangsloch 31b versehen, dessen mittlerer Teil, soweit er nicht in den Bereich des Längsloches 31a fällt, durch eine Bohrung gebildet ist, die den Schaft 19b des Stiftes 19 mit geringem radialen Spiel um dessen Längsachse drehbar und entlang von dieser verschiebbar führt. Die beiden Mündungen jedes Durchgangsloches 31b sind vorzugsweise mit einer konischen Ansenkung versehen. Ferner ist für jeden Stift 19 auf der der Achse des exzentrischen Längsloches 31a abgewandten Seite der Längsmittelachse des Trägers 31 in diesem eine rechtwinklig zu seiner Längsmittelachse verlaufenden Gewindebohrung 31c vorhanden. Der Stift 19 kann mit einer Klemmschraube 33 am Träger 31 festgeklemmt werden, die einen in die Gewindebohrung 31c eingeschraubten Gewindeteil und einen Kopf aufweist. An seinem dem Verbindungsglied 17 zugewandten Ende besitzt der Träger 31 einen Fortsatz 31d, der ungefähr ein halbkreisförmiges Profil hat und dadurch gebildet werden kann, dass man vom Endabschnitt des Trägers 31 entlang von dessen Achse ungefähr einen halben Zylinder wegschneidet. Wie noch erläutert wird, dient der Fortsatz 31d als Gelenkteil für das Gelenk 23 und weist eine ebene, entlang der Mittellängsachse des Trägers 31 und vorzugsweise durch diese verlaufende, zu den Achsen der Durchgangslöcher 31b rechtwinklige Auflagefläche 31e und ein rechtwinklig zu dieser und also parallel zu den Achsen der Durchgangslöcher 31b und der in diesen angeordneten Stifte 19 verlaufendes Durchgangsloch 31f auf. Dieses besteht aus einer abgestuften Bohrung mit einem engeren, in die Auflagefläche 31e mündenden Abschnitt und einem weiteren, in die im Profil ungefähr halbkreisförmige Aussenfläche des Fortsatzes 31d mündenden Abschnitt.

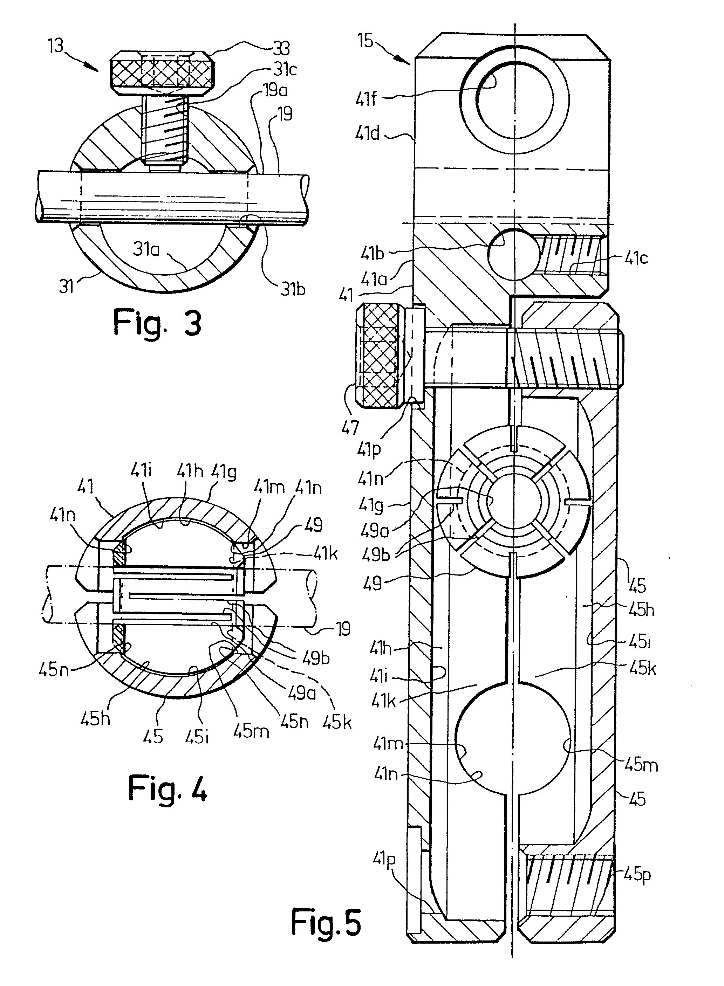

Nun soll anhand der Fig. 1, 2, 4 und 5 die Ausbildung des anderen Halters 15 erläutert werden, der einen einstückigen Träger 41 mit einem kurzen zylindrischen Abschnitt 41a mit einem quer zu seiner Achse verlaufenden Durchgangsloch 41b aufweist. Im letzteren ist einer der Stifte 19 in seiner Längsrichtung verschiebbar und drehbar gehalten und mit einer Klemmschraube 43 festgeklemmt, die in eine Gewindebohrung 41c des Trägers 41 eingeschraubt ist. Auf der dem Verbindungsglied 17 zugewandten Seite des Abschnittes 41a schliesst an diesen ein Fortsatz 41d an, der ähnlich wie der Fortsatz 31d ausgebildet ist und analog wie dieser eine ebene Auflagefläche 41e bildet und ein Durchgangsloch 41f besitzt. Die Auflagefläche 41e verläuft parallel zur Längsachse des Halters 15 und insbesondere des zylindrischen Abschnittes 41a und vorzugsweise direkt durch diese Längsachse und rechtwinklig zur Achse des Durchgangsloches 41f. An die dem Verbindungsglied 17 abgewandte Seite des zylindrischen Abschnittes 41a schliesst ein Fortsatz an, der einen Klemmteil 41g bildet und dessen Profil ein sich nicht ganz über einen halben Kreisumfang erstreckendes Kreissegment bildet. Der Klemmteil 41g spannt im Profil eine Kreissehne auf, die rechtwinklig zur Auflagefläche 41e verläuft und ist auf seiner im Profil durch die genannte Kreissehne begrenzten und

der Längsachse des Halters 15 zugewandten Seite mit einer länglichen Vertiefung 41h versehen. Diese wird durch eine im Profil konkav gebogene, innere Grundfläche 41i des Klemmteils 41g und durch zwei ebene, zueinander parallele Seitenflächen 41k begrenzt. Die diese bildenden, die Vertiefung 41h auf ihren einander abgewandten Längsseiten begrenzenden Seitenwände sind von zwei in der Längsrichtung des Klemmteils 41g voneinander beabstandeten, die Vertiefung 41h rechtwinklig kreuzenden Einschnitten 41m durchbrochen, die in ihrem Profil, d.h. in ihrer in der Fig. 5 ersichtlichen Ansicht, etwas weniger als 180° betragende Kreisbogen bilden. Der mittlere, innere Teil jedes Einschnittes 41m ist, soweit er nicht in den Bereich der Vertiefung 41h fällt und von dieser unterbrochen ist, durch eine einen Zylindersektor bildende Fläche begrenzt. Dort, wo diese die beiden Seitenflächen 41k schneidet, ergeben sich entlang einem Kreisbogen verlaufende Kanten. Wie noch erläutert wird, bilden diese Klemmbereiche 41n. An ihren in die einen Zylindersektor bildende Aussenfläche des Klemmteils 41g mündenden Enden sind die Einschnitte 41m durch geneigte, sich nach aussen erweiternde Flächen begrenzt, die durch einen Teil einer Konusfläche gebildet sind. Der Klemmteil 41g ist ferner noch mit zwei Durchgangslöchern 41p versehen, die durch abgestufte Bohrungen gebildet sind.

Der Halter 15 weist ferner einen zweiten, separaten Klemmteil 45 auf. Dieser ist durch einen länglichen, stabförmigen Körper gebildet, dessen Profil im allgemeinen die Form eines Kreissegmentes hat, das nicht ganz einen Halbkreis bildet. Dieser Klemmteil 45 ist geringfügig kürzer als der Klemmteil 41g. Die beiden Klemmteile 41g, 45 sind einander mit ihren im Profil durch eine Kreissehne gebildeten Seiten zugewandt und im Profil mindestens im wesentlichen bezüglich der zwischen ihnen verlaufenden Ebene symmetrisch zueinander. Der Klemmteil 45 weist eine durch eine gebogene Grundfläche 45i und ebene Seitenflächen 45k begrenzte Vertiefung 45h auf, die etwas kürzer als die Vertiefung 41h, aber im Profil symmetrisch zu dieser ist. Der Klemmteil 45 ist ferner mit symmetrisch zu den Einschnitten 41m angeordneten Einschnitten 45m versehen. Bei der Schnittstelle zwischen den Einschnitten 45m und den Seitenflächen 45k ergeben sich kreisbogenförmige Kanten, die zu den Klemmbereichen 41n symmetrische Klemmbereiche 45n bilden. Der Klemmteil 45 besitzt zwei Gewindebohrungen 45p, die in den die Vertiefung 45h bei ihren Enden begrenzenden Endabschnitten des Klemmteils 45 angeordnet sind und je mit einem Durchgangsloch 41p des Klemmteils 41g fluchten. Der Klemmteil 45 ist mit zwei durch die Durchgangslöcher 41p hindurch in die Gewindebohrungen 45p eingeschraubten Klemmschrauben 47, von denen in der Fig. 5 nur eine dargestellt ist, am Klemmteil 41g gehalten.

Zwischen den beiden Klemmteilen 41g und 45 sind im Bereich von deren einander paarweise zugewandten Einschnitten 41m bzw. 45m zwei Führungskörper 49 mit kugelförmigen oder, genauer gesagt, kugelschichtförmigen Aussenflächen angeordnet, wobei in den Fig. 2 und 5 nur ein Führungskörper 49 gezeichnet ist. Die kugel- oder kugelschichtförmigen Führungskörper 49 weisen ein durch ihr Zentrum verlaufendes Durchgangsloch 49a auf, das durch eine Bohrung gebildet ist, die an beiden Enden mit einer konischen Ansenkung versehen ist. Im Durchgangsloch 49a ist der Schaft von einem der Stifte 19 mit geringem radialem Spiel drehbar gelagert und in seiner Längsrichtung verschiebbar geführt. Jeder Führungskörper 49 ist mit einigen, beispielsweise mit acht über seinen Umfang verteilten, parallel zur Achse des Durchgangsloches 49a verlaufenden und von der Umfangsfläche des Führungskörpers 49 her radial in das Durchgangsloch 49a einmündenden Einschnitten 49b versehen. Die Einschnitte 49b sind etwas kürzer als das Durchgangsloch 49a, wobei entlang dem Umfang aufeinanderfolgende Einschnitte 49b abwechselnd bei den einander abgewandten Enden des Durchgangsloches beginnen. Der Führungskörper 49 wird daher durch die Einschnitte 49b in Sektoren unterteilt, die entlang dem Umfang abwechselnd auf verschiedenen Seiten durch Stege federnd miteinander verbunden sind.

Die Durchmesser der Führungskörper 49 sind derart bemessen, dass die beiden Klemmteile 41g, 45, wenn sie mit ihren Klemmbereichen 41n bzw. 45n an den Führungskörpern 49 anliegen, auf ihren im Profil durch eine Kreissehne begrenzten Seiten voneinander durch einen Spalt getrennt sind. Die Zentren der Führungskörper befinden sich in diesem Spalt, und zwar auf der Längsachse des Halters 15, die in der in den Fig. 1 und 2 gezeichneten Stellung mit der Längsachse 21 zusammenfällt. Wenn die beiden Klemmschrauben 47 lose sind, können die beiden je einen der Führungskörper 49 durchdringenden Stifte 19 um ihre Längsachse gedreht, in ihrer Längsrichtung verschoben und zusätzlich mitsamt den Führungskörpern 49 um deren Zentrum räumlich verschwenkt werden. Der Verschwenkungswinkel beträgt dabei, ausgehend von den in den Fig. 1, 2, 4 und 5 dargestellten Mittelstellungen der Stifte 19 und Führungskörper 49 auf alle Seiten hin mindestens 20° und beispielsweise mindestens oder ungefähr 25°. Wenn die beiden Klemmschrauben 47 festgezogen sind, werden die beiden Führungskörper 49 zwischen den Klemmteilen 41g, 45 zusammengedrückt und festgeklemmt und klemmen dann ihrerseits die ihnen angeordneten Stifte 19 fest. Da die Klemmteile 41g, 45 bei Klemmbereichen 41n bzw. 45n an den Führungskörpern 49 angreifen, die durch bogenförmige Kanten oder allenfalls sehr schmale, entlang von diesen verlaufenden Flächenstreifen gebildet sind, ergeben sich beim Zusammenklemmen dort relativ hohe Druckkräfte, die eine sichere Fixierung der Führungskörper in der gewählten Schwenkstellung gewährleisten.

Die Ausbildung der das Verbindungsglied 17 und die Gelenke 23, 25 bildenden Teile des Fixateurs 11 kann aus den Fig. 1, 2, 5, 6, 7, 8 und 9 ersehen werden. Das Verbindungsglied 17 weist eine Hülse 61 mit einem zylindrischen Hauptteil auf, dessen Längsachse in der in den Fig. 1 und 2 gezeigten Stellung mit der Längsachse 21 zusammenfällt und die an beiden Enden mit einem flanschartigen Kragen 61a bzw. 61b versehen ist, deren Aussendurchmesser mindestens ungefähr gleich dem Aussendurchmesser des Trägers 31 des Halters 13 und des zylindrischen

Abschnittes 41a des Halters 15 ist. An den sich näher beim Halter 13 befindenden Kragen 61a schliesst ein exzentrisch zur Hülsenachse angeordneter Fortsatz 61d an, dessen Aussenfläche mit einem Sektor der Umfangsfläche des Kragens 61a fluchtet und dessen freier Endabschnitt auf seiner der Hülsenachse zugewandten Seite eine ebene Auflagefläche 61e bildet sowie mit einer rechtwinklig zu dieser verlaufenden Gewindebohrung 61f versehen ist. Der Fortsatz 31d und der Fortsatz 61d bilden je einen Gelenkteil des Gelenkes 23, wobei die beiden Fortsätze mit ihren einander zugewandten Auflageflächen 31e, 61e aufeinander aufliegen. Ein schraubenartiger Gelenkbolzen 63 weist einen in die Gewindebohrung 61f eingeschraubten Gewindeteil, einen das Durchgangsloch 31f durchdringenden und den Halter 13 dort schwenkbar lagernden, zylindrischen Lagerteil und einen Kopf auf. Wenn der Gelenkbolzen 63 lose ist, kann der Halter 13 bezüglich des Verbindungsgliedes 17 um die parallel zu den Achsen der Durchgangslöcher 31b verlaufende Achse des Gelenkbolzens 63 verschwenkt werden. Ist hingegen der Gelenkbolzen 63 festgeschraubt, drückt die beim Übergang zwischen seinem Lagerteil und seinem Kopf vorhandene, durch eine Radialfläche des letzteren gebildete Schulterfläche den Fortsatz 31d gegen den Fortsatz 61d und klemmt diese Teile dadurch in der eingestellten Schwenkstellung fest.

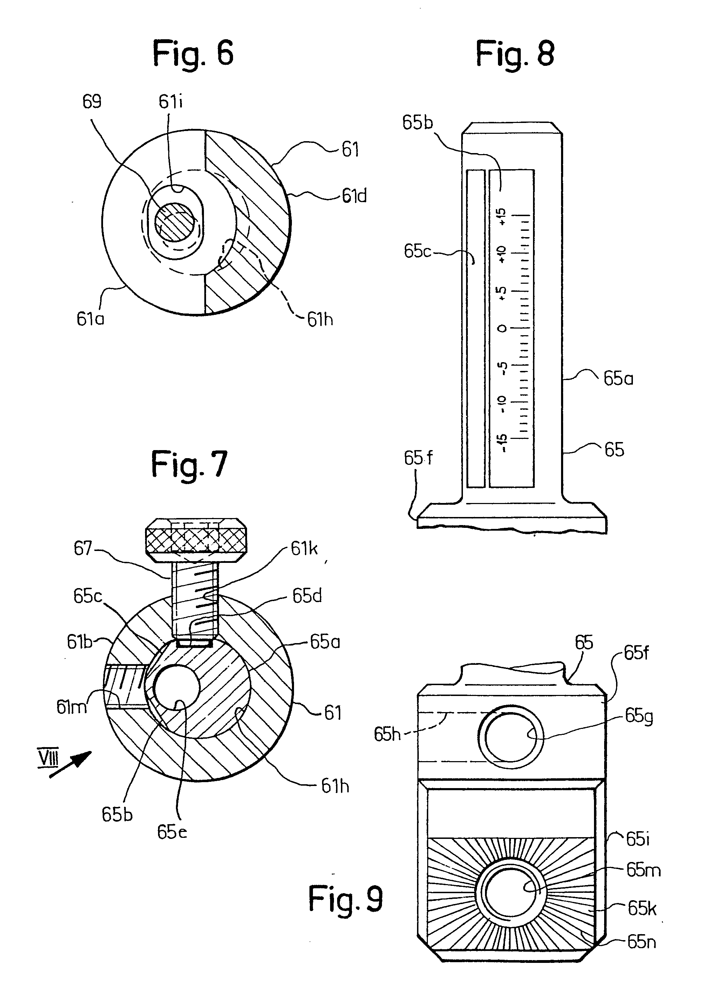

Die Hülse 61 weist eine durchgehende Längsöffnung 61g auf, deren Hauptteil durch einen zur Hülsenachse koaxialen, kreiszylindrischen Abschnitt 61h, d.h. eine Bohrung gebildet ist und in die dem Halter 15 zugewandten Stirnfläche des Kragens 61b mündet. Der dem Halter 13 zugewandte Endabschnitt 61i der Längsöffnung 61g ist zur Hülsenachse exzentrisch, und zwar auf deren dem Fortsatz 61d abgewandten Seite hin versetzt. Wie es aus der Fig. 6 ersichtlich ist, hat der Endabschnitt 61i im Querschnitt die Form eines länglichen Schlitzes, dessen längere Ausdehnung parallel zu der im Querschnitt vom Fortsatz 61d aufgespannten Kreissehne verläuft. Im übrigen liegt der Endabschnitt 61i in axialer Projektion vollständig innerhalb des zylindrischen Abschnittes 61h der Längsöffnung 61g und bildet also eine exzentrische Verengung von diesem. Im Bereich des dem Halter 15 zugewandten Kragens 61b ist die Hülse 61 mit zwei in der Fig. 7 ersichtlichen Gewindebohrungen 61k und 61m versehen, von denen die letztere auch in der Fig. 2 sichtbar ist. Die beiden Gewindebohrungen 61k, 61m münden beide in zur Hülsenachse radialer Richtung in den zylindrischen Abschnitt 61h der Längsöffnung 61g und sind entlang des Hülsenumfangs gegeneinander versetzt, und zwar beispielsweise um einen Bogen oder Winkel von 90°, wobei ihre Achse in der gleichen, in der Fig. 7 dargestellten, zur Hülsenachse rechtwinkligen Schnittebene liegen und miteinander einen Winkel bilden.

Ein besonders deutlich in den Fig. 2, 7, 8 und 9 ersichtlicher Dorn 65 weist einen Abschnitt 65a mit einer teilweisen kreiszylindrischen Umfangsfläche auf, der mit geringem radialen Spiel um die Hülsenachse dreh- oder schwenkbar im zylindrischen Abschnitt 61h der Hülsen-Längsöffnung 61g gelagert und axial verschiebbar geführt ist. Die Umfangsfläche des Dornabschnittes 65a ist mit drei Ausnehmungen versehen, die entlang des Dornumfangs gegeneinander versetzte, parallel zur Dornachse verlaufende, Klemm-Flächen 65b, 65d bilden und etwas kürzer sind als der Dornabschnitt 65a. Die beiden Klemm-Flächen 65b und 65c sind eben und bilden in dem in der Fig. 7 dargestellten Querschnitt Kreissehnen des Dornabschnittes 65a, während die Klemm-Fläche 65d durch den ebenen Boden einer Nut gebildet ist, deren seitliche Längsflächen rechtwinklig zur Klemm-Fläche 65d verlaufen. Die drei Klemm-Flächen 65b, 65c, 65d sind beispielsweise ungefähr über den halben Umfang des Dornabschnittes 65a verteilt und die aufeinanderfolgenden Klemm-Flächen bilden paarweise zusammen Winkel, nämlich Winkel von 120°, wobei zwischen den einander benachbarten Klemm-Flächen jeweils noch ein schmaler Streifen der zylindrischen Umfangsfläche vorhanden ist. Eine Klemmschraube 67 weist einen Kopf sowie einen Gewindeteil auf, der an seinem dem Kopf abgewandten Ende mit einem dünneren, zylindrischen Ansatz versehen ist und wahlweise in eine der Gewindebohrungen 61k oder 61m eingeschraubt werden kann. Im gezeichneten Zustand ist die Klemmschraube 67 in die Gewindebohrung 61k eingeschraubt und ihr Ansatz ragt mit nur sehr geringem seitlichem Spiel in die Nut hinein, deren Boden die Klemm-Fläche 65d bildet, so dass der Ansatz bei nur lose eingeschraubter Klemmschraube 67 den Dorn 65 beim Verschieben führt und gegen Verdrehungen sichert. Wenn die Klemmschraube hingezogen festgeschraubt ist, greift die ebene Stirnfläches ihres Ansatzes an der Klemm-Fläche 65d des Dorns an und klemmt diesen in der gezeichneten Schwenk- und Schiebestellung fest, wobei diese Schwenkstellung im folgenden als Mittel-Schwenkstellung bezeichnet wird. Wenn man die Klemmschraube 67 aus der Gewindebohrung 61k herausschraubt, kann man den Dorn ausgehend von seiner Mittel-Schwenkstellung wahlweise um 30° in der einen oder anderen Schwenkrichtung um seine Längsachse verschwenken, die Klemmschraube 67 in die Gewindebohrung 61m einschrauben und mit dieser den Dorn je nach Schwenkstellung entweder bei der Klemm-Fläche 65b oder 65c festklemmen. Der Dorn kann also wahlweise in drei verschiedenen Schwenkstellungen und in kontinuierlich einstellbaren Schiebestellungen festgeklemmt werden.

Der Dorn 65 kann beispielsweise bei seiner Klemm-Fläche 65b mit einer in seiner Längsrichtung verlaufenden Skala versehen sein, deren sich gerade bei der Stirnfläche des Kragens 61b befindende Markierung die Schiebestellung des Dorns kennzeichnet. Die Skala kann dabei ausgehend von einer Null-Markierung in den beiden einander entgegengesetzten Längsrichtungen mit Masszahlen versehen sein und beispielsweise eine Millimeter-Teilung aufweisen.

Der Dorn 65 ist ferner mit einem zu seiner Längsachse parallelen, aber exzentrischen, in der Fig. 2 ersichtlichen Längsloch 65e versehen, das als Durchgangsloch oder eventuell als in die Endfläche des Dornabschnittes 65a mündendes Sackloch ausgebildet sein kann. Mindestens ein Teil dieses Längsloches 65e, und zwar zweckmässigerweise mindestend sein dem Halter 13 zugewandter Endabschnitt, ist mit einem Innengewinde versehen, während der restliche Teil des Längsloches 65e einen Durchmesser aufweist, der etwas grösser als der Gewindedurchmesser ist. Eine Spannschraube 69 weist einen in das Innengewinde des Längsloches 65e eingeschraubten Gewindeteil, einen den exzentrischen Endabschnitt 61i der Längsöffnung 61g der Hülse 61 durchdringenden Schaft oder Gewindeteilabschnitt und einen Kopf auf. Die schlitzförmige Ausbildung des vom Schaft der Spannschraube 69 durchdrungenen Endabschnittes 61i der Längsöffnung 61g und das Spiel des Schraubenschaftes in dieser gewährleistet, dass die Spannschraube 69 trotz ihrer exzentrischen Anordnung im Dorn 65 dessen wahlweise Verschwenkung in eine der drei vorgängig erwähnten Schwenkstellungen ermöglicht. Der Kopf der Spannschraube 69 befindet sich zwischen der Stirnfläche des Kragens 61a und der Auflagefläche 61e des Fortsatzes 61d und ragt teilweise in eine im letzteren vorhandene Ausnehmung 61n hinein.

Wenn man die Spannschraube 69 tiefer in den Dorn 65 einschraubt, liegt die von ihrem Kopf gebildete, radiale Schulterfläche an der dem Halter 13 zugewandten, zur Achse der Hülse 61 rechtwinkligen Fläche des Kragens 61a an. Bei gelöster Klemmschraube 67 kann der Dorn 65 durch Einschrauben der Spannschraube 69 in diesen tiefer in die Hülse 61 hineingezogen werden. Dadurch kann eine zur Längsachse des Verbindungsgliedes 17 parallele Kraft erzeugt werden, die die beiden Halter 13, 15 gegeneinander zieht und die beiden Knochenstücke 1a, 1b gegeneinander spannt und ihre Frakturfläche 1c bzw. 1d aneinander andrückt.

An das aus der Hülse 61 herausragende Ende des Dornabschnittes 65a schliesst eine zylindrische Verdickung 65f an, deren Durchmesser mindestens ungefähr gleich denjenigen des Trägers 31 und der Krägen 61a, 61b ist. Die Verdickung 65f ist mit einem Durchgangsloch 65g versehen, in dem einer der Stifte 19 drehbar gehalten sowie verschiebbar geführt ist. In einer quer zum Durchgangsloch 65g verlaufenden, in diese mündenden Gewindebohrung 65h ist eine Klemmschraube 71 eingeschraubt, mit der der betreffende Stift 19 festklemmbar ist. Das dem Halter 15 zugewandte Ende des Dorns 65 wird durch einen Fortsatz 65i gebildet, der eine an der Auflagefläche 41e des Fortsatzes 41d anliegende Auflagefläche 65k und eine Gewindebohrung 65m aufweist. Wenn sich der Dorn 65 bezüglich der Hülse 61 in der gezeichneten Mittel-Schwenkstellung befindet, verläuft die ebene Auflagefläche 65k parallel zur ebenen Auflagefläche 61e und liegt sogar in der gleichen Ebene wie diese. Ein analog zum Gelenkbolzen 63 ausgebildeter Gelenkbolzen 73 weist einen in die Gewindebohrung 65m eingeschraubten Gewindeteil, einen das Durchgangsloch 41f durchdringenden und den Halter 15 bezüglich des Verbindungsgliedes 17 schwenkbar lagernden Lagerteil und einen Kopf auf, mit dem die die Gelenkteile des Gelenkes 25 bildenden Fortsätze 41d und 65i aneinander angedrückt und festgeklemmt werden können.

Die paarweise aneinander anliegenden Auflageflächen 31e, 61e und 41e, 65k sind mit einem Kranz von abwechselnd aufeinanderfolgenden Zähnen und Rillen versehen, die sich entlang von Geraden erstrecken, die radial von den durch die Gelenkbolzen 63, 73 definierten Schwenkachsen wegverlaufen. Diese Zähne und Rillen bilden Verzahnungen, von denen in der Fig. 9 die Verzahnung der Auflagefläche 65k dargestellt und mit 65h bezeichnet ist. Wenn die Auflagefläche 31e, 61e und 41e, 65k durch Festschrauben der Gelenkbolzen gegeneinander gedrückt werden, greifen diese Verzahnungen ineinander ein, so dass die Gelenke 23, 25 im festgeklemmten Zustand auch beim Auftreten von sehr grossen Kräften und Drehmomenten starr fixiert bleiben. Die Verzahnungen sind relativ fein und jede kann beispielsweise etwa 120 Zähne aufweisen, so dass die Gelenke 23, 25 trotz der Verzahnungen in quasi kontinuierlich aufeinanderfolgenden Schwenkstellungen fixierbar sind.

Die Köpfe der verschiedenen Klemmschrauben 33, 43, 47, 67 und Gelenkbolzen 63, 73 haben vorteilhafterweise Umfangsflächen, die mindestens in einem axialen Teilbereich mit einer Randrierung oder dergleichen versehen sind. Ferner weisen die Köpfe der Klemmschrauben und Gelenkbolzen auf ihre Stirnseiten ein Sechskantloch zum Angreifen mit einem Inbusschlüssel auf. Der Kopf der Spannschraube 69 ist ebenfalls mindestens in einem axialen Teilbereich mit einer Randrierung versehen und weist ferner mindestens ein von der Seite her zugängliches Loch, beispielsweise eine diametrale Durchgangsbohrung zum Einführen eines Schlüssels mit einem entsprechenden Zapfen auf.

Die beschriebenen Teile des Fixateurs können alle aus Metall, beispielsweise rostfreiem Stahl oder einer Titanlegierung, bestehen.

Der Fixateur befindet sich vor seiner Verwendung zweckmässigerweise in einem Zustand, in welchem die beiden Halter 13, 15 und das Verbindungsglied 17 entlang der gemeinsamen Längsachse 21 verlaufen, sich der Dorn 65 in der gezeichneten Mittel-Schwenkstellung befindet und die Längsachsen von allen zum Führen von Stiften 19 dienenden Durchgangslöchern 31b, 41b, 49a, 65g in der gleichen Ebene liegen sowie parallel zueinander verlaufen. Ferner ist der Dorn 65 bezüglich der Hülse 61 vorzugsweise in einer mittleren Schiebestellung fixiert, bei der sich die Null-Markierung der auf der Klemm-Fläche 65b vorhandenen Skala bei der Stirnfläche des Kragens 61b befindet. Wenn nun der Fixateur 11 zur Fixierung eines Knochens benutzt werden soll, kann man beispielsweise zuerst das Knochenstück 1a mit zwei in den Halter 13 eingesetzten Stiften 19 und das Knochenstück 1b mit zwei in die Führungskörper 49 des Halters 15 eingesetzten Stiften 19 provisorisch fixieren. Dies kann bei einer durch Unfall entstandenen Knochenfraktur beispielsweise schon am Unfallort geschehen. Wenn es beim danach erfolgenden Justieren der Knochenstücke erforderlich ist, die vom Halter 15 gehaltenen Stifte 19 bezüglich der vom Halter 13 gehaltenen Stifte um die Längsachse 21 zu verschwenken, kann man die Klemmschrauben 47 vorübergehend lösen und die Führungskörper 49 entsprechend verschwenken. Falls dies nicht ausreichen sollte, kann man zusätzlich den Dorn 65 ausgehend von seiner Mittel-Schwenkstellung bezüglich der Hülse 61 verschwenken und in einer der anderen vorgesehenen Schwenkstellungen fixieren. Ferner kann man natürlich den Halter 13 bezüglich der Hülse 61 um den Gelenkbolzen 63 herum und den Halter 15 bezüglich des Dorns 65 um den Gelenkbolzen 73 herum verschwenken und in verschiedenen Schwenkstellungen fixieren. Wenn die Knochenstücke 1a, 1b gerichtet sind, kann man mittels der Spannschraube 69 bei vorübergehend gelöster Klemmschraube 67 noch eine die Frakturflächen 1c bzw. 1d gegeneinander drückende Kraft erzeugen.

Im übrigen kann man, beispielsweise im Verlauf des Justiervorganges, bei Bedarf auch mehr als die vier erwähnten Stifte 19 in den Fixateur einsetzen. Wenn der Knochen beispielsweise in drei Stücke gebrochen ist, kann man eventuell das mittlere Knochenstücke mit mindestens einem der beiden Stifte 19 fixieren, die in der Dorn-Verdickung 65f bzw. im Abschnitt 41a des Trägers 41 festklembar sind. Es sei in diesem Zusammenhang noch darauf hinzuweisen, dass die beiden Halter 13 und 15 in vertauschten Stellungen angeordnet werden können.

Ferner kann auch ein Satz von Haltern 13, 15 und Verbindungsgliedern 17 und weiteren Fixateur-Komponenten bereitgestellt werden, so dass ein Chirurg im Bedarfsfall an jedem Ende eines Verbindungsgliedes 17 einen Halter 15 angelenken kann, der Stifte 19 räumlich verschwenkbar halten kann. Wenn der Chirurg über einen solchen Satz von Fixateur-Komponenten verfügt, kann er in einfachen Fällen eventuell auch einen Fixateur zusammensetzen, der an beiden Enden des Verbindungsgliedes 17 einen gemäss dem Halter 13 ausgebildeten Halter ohne räumlich verschwenkbare Stifte aufweist.

Es sei hier noch darauf hingewiesen, dass der Fixateur nicht nur zum Fixieren durch eine Knochenfraktur entstandener Knochenstücke, sondern auch zum Fixieren von Knochenstücken verwendet werden kann, die durch eine Osteotomie, d.h. eine chirurgisch vorgenommene Durchtrennung gebildet wurden. Nach einer Osteotomie ist es häufig notwendig, den Knochen zu distrahieren, d.h. zu strecken, und die Knochenstücke, wenn sie wieder mehr oder weniger miteinander verwachsen sind, mit voneinander weggerichteten Kräften zu beaufschlagen. Eine derartige Streckung des Knochens lässt sich ebenfalls mit dem Fixateur 11 erzielen, indem die Spannschraube 69 im Verlauf des Behandlungs- und Heilungsprozesses bei jeweils vorübergehend und gelöster Klemmschraube 67 ein wenig losgeschraubt, d.h. um eine gewisse Distanz aus dem Dorn 65 herausgeschraubt wird. Der Kopf der Spannschraube 69 greift dann an der dem Kragen 61a abgewandten Begrenzungsfläche der Ausnehmung 61a an, wodurch der Dorn 65 aus der Hülse 61 heraus und die beiden Gelenke 23, 25 sowie natürlich auch die beiden Halter 13, 15 voneinander weggedrückt werden, und zwar bei allen vorgesehenen Schwenkstellungen des am Fortsatz 61d ausgelenkten Halters 13.

Der Fixateur ermöglicht also eine Justierung der durch eine Fraktur oder eine Osteotomie entstandenen Knochenstücke mit vielen Verstell-Freiheitsgraden. Wenn die verstellbaren Teile des Fixateurs fixiert sind, ist dieser aber trotz der vielen Verstellmöglichkeiten sehr stabil. Dabei ist insbesondere von Nutzen, dass jeder der räumlich verschwenkbaren Führungskörper 49 nur die Kraft eines einzigen Stiftes übertragen muss und sich relativ nahe bei der Stelle befindet, bei der der Knochen am betreffenden Stift angreift. Im übrigen ist der Fixateur verhältnismässig kompakt und lässt sich leicht handhaben. Gegenüber dem bereits in der Einleitung erwähnten, aus der Schweizer Patentschrift 608 712 bekannten Fixateur, bei dem den Knochen durchdringende Stifte auf beiden Seiten von diesem mit Haltern fixiert werden müssen, ergibt der erfindungsgemässe Fixateur 11 für einen Patienten den wesentlichen Vorteil, dass die Stifte nur auf einer Seite des Knochens mit ausserhalb des den Knochen enthaltenden Körperteils angeordneten Mitteln gehalten werden müssen. Der erfindungsgemässe Fixateur 11 kann zudem mit relativ geringem Zeitaufwand zerlegt und gereinigt werden, was insbesondere deshalb von Nutzen ist, weil er abgesehen von den Stiften für den mehrmaligen Gebrauch vorgesehen ist.

Der Fixateur kann natürlich in verschiedener Hinsicht modifiziert werden.

Beispielsweise könnten die zum Führen von Stiften 19 dienenden Löcher 41b, 65g wegfallen. Ferner kann auch die Anzahl der vom Halter 13 gehaltenen Stifte und die Anzahl der vom Halter 15 verschiebbar und räumlich verschwenkbar gehaltenen Stifte variieren. Der Halter 13 soll aber mindestens einen und vorzugsweise mindestens zwei Stifte halten und der Halter 15 soll mindestens einen Stift und vorzugsweise mindestens zwei Stifte räumlich verschwenkbar halten. Abgesehen von der bereits erwähnten Möglichkeit, einen Satz von Fixateur-Komponenten bereitzustellen, die dem Chirurgen die Auswahl der Halter ermöglichen, könnte natürlich auch gleich ein fertiger Fixateur bereitgestellt werden, bei dem beispielsweise der Halter 13 durch einen Halter ersetzt ist, der ausgebildet ist, um mindestens einen Stift räumlich verschwenkbar zu halten.

Der Abschnitt 65a des Dorns 65 könnte anstelle der drei Klemm-Flächen 65b, 65c, 65d mit anderen Klemm-Flächen versehen sein, die nicht rotationssymmetrisch zur Dornachse sind, d.h. im Querschnitt keine konvexen, um die Dornachse herum verlaufenden Kreisbogen bilden. Anstelle der drei gezeichneten Klemm-Flächen 65b, 65c, 65d könnten beispielsweise im Querschnitt konkav gebogene Klemm-Flächen vorhanden sein. Ferner könnte die Anzahl Klemm-Flächen auch grösser oder kleiner als drei sein. Eventuell könnte sogar nur eine einzige Klemm-Fläche vorhanden sein, wobei dafür beispielsweise die Hülse 61 etwa drei oder mehr entlang ihrem Umfang versetzte Gewindebohrungen aufweisen könnte, in die wahlweise eine Klemmschraube zum Festklemmen des Dorns eingesetzt werden könnte. Wenn mindestens zwei Klemm-Flächen der genannten Art vorhanden sind, könnte andererseits die Hülse natürlich auch nur eine einzige Gewindebohrung mit einer zum Festklemmen des Dorns dienenden Klemmschraube aufweisen.

Der den Kragen 61a durchdringende Endabschnitt 61i der Längsöffnung 61g könnte statt durch einen geraden Schlitz auch durch einen um die Hülsenachse herum gebogenen Schlitz gebildet sein.

Die kugel- oder kugelschichtförmigen Führungskörper könnten anstelle von Einschnitten eventuell auch nur Schlitze aufweisen, deren beiden Enden durch Stege des Führungskörpers begrenzt sind.

Im übrigen könnten an den in einen Knochen eingeschraubten Stiften noch zusätzlich Fixierelemente befestigt werden, falls dies für besondere Zwecke wünschenswert ist.

**Patentansprüche**

1. Fixateur zum Fixieren von Knochenstücken (1a, 1b), mit zwei länglichen Haltern (13, 15), in denen zum Eingreifen in die Knochenstücke (1a, 1b) bestimmte Stifte (19) in ihrer eigenen Längsrichtung verstellbar festklemmbar sind, und einem länglichen Verbindungsglied (17), das bei einander abgewandten Enden durch je ein in verschiedenen Schwenkstellungen fixierbares Gelenk (23, 25) mit dem ihm zugewandten Ende eines der Halter (13, 15) verbunden ist, dadurch gekennzeichnet, dass jedes dieser beiden Gelenke (23, 25) einen eine zur Längsrichtung des jeweiligen Halters (13, 15) und des Verbindungsgliedes (17) rechtwinklige Schwenkachse definierenden Gelenkbolzen (63, 73) aufweist und dass mindestens ein Halter (15) mit Halte- und Klemm-Mitteln (41, 45, 47) mindestens einen Führungskörper (49), der einen der Stifte (19) verschiebbar führt, räumlich verschwenkbar und in verschiedenen Schwenkstellungen festklemmbar hält.

2. Fixateur nach Anspruch 1, dadurch gekennzeichnet, dass die Halte- und Klemm-Mittel (41, 45, 47) zwei Klemmteile (41g, 45) und mindestens eine zum Gegeneinanderspannen von diesen dienende Klemmschraube (47) aufweisen, dass die Klemmteile (41g, 45) auf ihren einander zugewandten Seiten mit Vertiefungen (41h, 45h) versehen sind, in denen der bzw. mindestens einer der Führungskörper (49) gehalten ist, und dass der bzw. jeder Führungskörper (49) eine kugel- oder kugelschichtförmige Aussenfläche und mindestens einen Einschnitt (49b) oder Schlitz aufweist und mit den Klemmteilen (41g, 45) festklemmbar sowie derart zusammendrückbar ist, dass er in festgeklemmtem und zusammengedrücktem Zustand seinerseits den ihn durchdringenden Stift (19) festklemmt.

3. Fixateur nach Anspruch 2, dadurch gekennzeichnet, dass die beiden Klemmteile (41g, 45) mindestens zwei je einen Stift (19) verschiebbar führende Führungskörper (49) der genannten Art räumlich verschwenkbar und in verschiedenen Schwenkstellungen festklemmbar halten und dass zum Gegeneinanderspannen der beiden Klemmteile (41g, 45) zwei Klemmschrauben (47) vorhanden sind, zwischen denen sich die Führungskörper (49) befinden.

4. Fixateur nach einem der Ansprüche 1 bis 3, dadurch gekennzeichnet, dass die einander abgewandten Enden des Verbindungsgliedes (17) zueinander um eine zur Längsrichtung des Verbindungsgliedes (17) parallele Achse verschwenkbar und in verschiedenen Schwenkstellungen fixierbar sind.

5. Fixateur nach einem der Ansprüche 1 bis 4, dadurch gekennzeichnet, dass das Verbindungsglied (17) eine Hülse (61) mit einer Längsöffnung (61g)

und einen verschwenkbaren sowie in verschiedenen Schwenkstellungen in dieser festklemmbaren Dorn (65) aufweist und dass das eine Gelenk (23) den einen Halter (13) mit der Hülse (61) und das andere Gelenk (25) den anderen Halter (15) mit dem Dorn (65) verbindet.

6. Fixateur nach Anspruch 5, dadurch gekennzeichnet, dass die Hülse (61) mit mindestens einer Klemmschraube (67) zum Angreifen an einer nicht zur Dornachse rotationssymmetrischen, beispielsweise ebenen und/oder durch eine Nut gebildeten oder im Querschnitt konkav gebogenen Klemm-Fläche (65b, 65c, 65d) des Dorns (65) und zum Festklemmen des letzteren versehen ist, wobei die Hülse (61) mindestens zwei entlang ihrem Umfang gegeneinander versetzte, qzer zu ihrer Längsachse verlaufende, in ihre Längsöffnung (61g) mündende Gewindebohrungen (61k, 61m) zum Halten der Klemmschraube (67) und/oder der Dorn (65) mindestens zwei entlang seinem Umfang gegeneinander versetzte, nicht zur Dornachse rotationssymmetrische Klemm-Flächen (65b, 65c, 65d) aufweist, an denen die Klemmschraube (67) wahlweise angreifen kann.

7. Fixateur nach einem der Ansprüche 1 bis 6, dadurch gekennzeichnet, dass das Verbindungsglied (17) eine Hülse (61) mit einer Längsöffnung (61g) und einen axial verschiebbar in dieser geführten, in verschiedenen Schiebestellungen festklemmbaren Dorn (65) aufweist, dass das eine Gelenk (23) den einen Halter (13) mit der Hülse (61) und das andere Gelenk (25) den anderen Halter (15) mit dem Dorn (65) verbindet, dass der Dorn (65) ein Längsloch (65e) aufweist, von dem mindestens ein Teil mit einem Innengewinde versehen ist, und dass eine in dieses eingeschraubte Spannschraube (69) mit einem von der Aussenseite der Hülse (61) her zugänglichen Kopf vorhanden ist, um den Dorn (65) gegen das die Hülse (61) mit dem Halter (13) verbindende Gelenk (23) und dadurch die beiden Halter (13, 15) gegeneinander zu ziehen.

8. Fixateur nach Anspruch 7, dadurch gekennzeichnet, dass die Hülse (61) am einen Ende einen bezüglich ihrer Längsachse exzentrischen Fortsatz (61d) aufweist, der zusammen mit einem Abschnitt des einen Halters (13) und dem einen Gelenkbolzen (63) das die Hülse (61) mit dem einen Halter (13) verbindende Gelenk (23) bildet, und dass die Spannschraube (69) zur Achse des den Dorn (65) führenden Abschnittes (61h) der Hülsen-Längsöffnung (61g) exzentrisch ist und einen Endabschnitt (61i) der Hülsen-Längsöffnung (61g) durchdringt, der auf die dem Fortsatz (61d) abgewandte Seite der Achse des den Dorn (65) führenden Abschnittes (61h) der Hülsen-Längsöffnung (61g) versetzt ist, wobei der Fortsatz (61d) vorzugsweise eine Ausnehmung (61n) aufweist, in die der Kopf der Spannschraube (69), vorzugsweise mit axialem Spiel, eingreift, so dass durch Losschrauben der Spannschraube (69) eine den Dorn (65) vom Fortsatz (61d) wegdrückende Kraft erzeugt werden kann.

9. Fixateur nach einem der Ansprüche 1 bis 8, dadurch gekennzeichnet, dass das Verbindungsglied (17) Führungs- und Klemm-Mittel (65g, 65h, 71) aufweist, um mindestens einen Stift (19) in dessen Längsrichtung verstellbar festzuklemmen, wobei diese Führungs- und Klemm-Mittel (65g, 65h, 71) den Stift (19) vorzugsweise räumlich unverschwenkbar parallel zur Schwenkachse von mindestens einem der beiden Gelenke (23, 25) verschiebbar führen.

10. Fixateur nach einem der Ansprüche 1 bis 9, dadurch gekennzeichnet, dass der Gelenkbolzen (63, 73) jedes Gelenks (23, 25) einen Gewindeteil, einen Lagerteil und einen Kopf aufweist und dass das Gelenk (23, 25) durch Festschrauben des betreffenden Gelenkbolzens (63, 73) blockierbar ist.

11. Fixateur nach einem der Ansprüche 1 bis 10, dadurch gekennzeichnet, dass die Halter (13, 15) und das Verbindungsglied (17) bei den Gelenken (23, 25) paarweise aufeinander aufliegende Auflageflächen (31e, 41e, 61e, 65k) mit Verzahnungen (65n) aufweisen, deren Zähne um die Schwenkachse des betreffenden Gelenks (23, 25) herum verteilt sind, so dass sie in verschiedenen Schwenkstellungen der Auflageflächen (31e, 41e, 61e, 65k) ineinander eingreifen können.

12. Fixateur nach einem der Ansprüche 1 bis 11, dadurch gekennzeichnet, dass der eine Halter (13) einen Träger (31) mit einem im allgemeinen kreiszylindrischen Abschnitt aufweist, der mit einem exzentrischen Längsloch (31a) und für jeden von diesem Halter (13) gehaltenen Stift (19) mit einem das Längsloch (31a) kreuzenden, den betreffenden Stift (19) parallel zur Schwenkachse des diesen Halter (13) mit dem Verbindungsglied (17) verbindenden Gelenks (23) verschiebbar sowie um die Stiftachse drehbar, aber bezüglich des Halters (13) räumlich unverschwenkbar führenden Durchgangsloch (31b) und mit einer in dieses mündenden Gewindebohrung (31c) zum Halten einer Klemmschraube (33) versehen ist, wobei sich die Gewindebohrung (31c) auf der der Längsloch-Achse abgewandten Seite der Achse (21) des im allgemeinen zylindrischen Träger-Abschnitts befindet.

13. Fixateur nach einem der Ansprüche 1 bis 12, dadurch gekennzeichnet, dass jeder der beiden Halter Halte- und Klemm-Mittel (41, 45, 47) der genannten Art sowie mindestens einen räumlich verschwenkbaren, in verschiedenen Schwenkstellungen festklemmbaren, einen Stift (19) verschiebbar führenden Führungskörper (49) der genannten Art aufweist.

## Claims

1. Setting device, for the setting of bone pieces (1a, 1b), with two elongate holders (13, 15), in which pins (19) intended for engagement into the bone pieces (1a, 1b) are resettable in their own longitudinal direction and clampable fast, and an elongate connecting member (17), which at mutually remote ends is connected with the end facing it of one of the holders (13, 15) through a respective hinge joint (23, 25) fixable in different pivotal positions, characterised thereby, that each of both these hinge joints (23, 25) displays a hinge pin (63, 73) defining a pivot axis at right angles to the longitudinal direction of the respective holder (13, 15) and of the connecting member (17) and that at least one holder (15) with retaining and clamping means (41, 45, 47) retains at least one guide body (49), which guides one of the pins (19) displaceably, to be pivotable in three dimensions and clampable fast in different pivotal settings.

2. Setting device according to claim 1, characterised thereby, that the retaining and clamping means (41, 45, 47) display two clamping parts (41g, 45) and at least one clamping screw (47) serving to tighten these together, that the clamping parts (41g, 45) are provided on their mutually facing sides with depressions (41h, 45h), in which the or at least one guide body (49) is retained, and that the or each guide body (49) displays an external surface in the shape of a sphere or of a spherical zone and at least one notch (49b) or slot and is clampable fast as well as compressible by the clamping parts (41g, 45) in such a manner that it in the clamped and compressed state in its turn clamps fast the pin (19) penetrating it.

3. Setting device according to claim 2, characterised thereby, that both the clamping parts (41g, 45) each retain at least two guide bodies (49) of the named kind, which displaceably guide a respective pin (19), to be pivotable in three dimensions and clampable fast in different pivotal settings and that two clamping screws (47), between which the guide bodies (49) are disposed, are present for the tightening together of both the clamping parts (41g, 45).

4. Setting device according to one of claims 1 to 3, characterised thereby, that the mutuallyl remote ends of the connecting member (17) are pivotable about an axis parallel to the longitudinal direction of the connecting member (17) and fixable in different pivotal settings.

5. Setting device according to one of claims 1 to 4, characterised thereby, that the connecting member (17) displays a sleeve (61) with a longitudinal opening (61g) and a stud (65), which is pivotable as well as clampable fast therein in different pivotal settings, and that the one hinge joint (23) connects the one holder (13) with the sleeve (61) and the other hinge joint (25) connects the other holder (15) with the stud (65).

6. Setting device according to claim 5, characterised thereby, that the sleeve (61) is provided with at least one clamping screw (67) for engagement at a clamping surface (65b, 65c, 65d), which is not rotationally symmetrical to the axis of the stud and is for example planar and/or formed by a groove or concavely curved in cross-section, of the stud (65) and for the firm clamping of the latter, wherein the sleeve (61) displays at least two threaded bores (61k, 61m), which are one displaced relative to the other along its circumference, extend transversely to its longitudinal axis and which open into its longitudinal opening (61g), for the retention of the clamping screw (67) and/or the stud (65) displays at least two clamping surfaces (65b, 65c, 65d), which are one displaced relative to the other along its circumference, are not rotationally symmetrical to the axis of the stud and at which the clamping screw (67) can engage selectably.

7. Setting device according to one of claims 1 to 6, characterised thereby, that the connecting member (17) displays a sleeve (61) with a longitudinal opening (61g) and a stud (65), which is guided in this to be axially displaceable and firmly clampable in different slide settings, that the one hinge joint (23) connects the one holder (13) with the sleeve (61) and the other hinge joint (25) connects the other holder (15) with the stud (65), that the stud (65) displays an elongate hole (65e), of which at least a part is provided with an internal thread, and that a tightening screw (69) which is threaded into this, with a head accessible from the outside of the sleeve (61) is present to draw the stud (65) against the hinge joint (23) connecting the sleeve (61) with the one holder (13) and thereby both the holders (13, 15) each against the other.

8. Setting device according to claim 7, characterised thereby, that the sleeve (61) at one end displays a prolongation (61d), which is eccentric with respect to its longitudinal axis and together with one portion of the one holder (13) and the one hinge pin (63) forms the hinge joint (23) connecting the sleeve (61) with the one holder (13), and that the tightening screw (69) is eccentric to the axis of that portion (61h) of the longitudinal sleeve opening (61g), which guides the stud (65), and penetrates an end portion (61i) of the longitudinal sleeve opening (61g), which portion is displaced to the side, which is remote from the prolongation (61d), of the axis of that portion (61h) of the longitudinal sleeve opening (61g), which guides the stud (65), wherein the prolongation (61d) preferably displays a recess (61n), into which the head of the tightening screw (69) engages, preferably with axial play, so that a force pressing the stud (65) away from the prolongation (61d) can be produced by unscrewing of the tightening screw (69).

9. Setting device according to one of claims 1 to 8, characterised thereby, that the connecting member (17) displays guiding and clamping means (65g, 65h, 71) to clamp fast at least one pin (19) resettable in its longitudinal direction, wherein these guiding and clamping means (65g, 65h, 71) guide the pin (19), preferably not to be pivotable in three dimensions, parallelly to the pivot axis of at least one of both the hinge joints (23, 25).

10. Setting device according to one of claims 1 to 9, characterised thereby, that the hinge pin (63, 73) of each hinge joint (23, 25) displays a threaded part, a bearing part and a head and that the hinge joint (23, 25) is blockable through screwing-tight of the hinge pin (63, 73) concerned.

11. Setting device according to one of claims 1 to 10, characterised thereby, that the holders (13, 15) and the connecting member (17) at the hinge joints (23, 25) display bearing surfaces (31e, 41e, 61e, 61k), which lie one against the other in pairs, with toothings (65n), the teeth of which are distributed around the pivot axis of the hinge joint (23, 25) concerned so that they can engage one into the other in different pivotal settings of the bearing surfaces (31e, 41e, 61e, 61k).

12. Setting device according to one of claims 1 to 11, characterised thereby, that the one holder (13) displays a carrier (31) with a generally circularly cylindrical portion which is provided with an eccentric elongate hole (31a) and, for each pin (19) retained by this holder (13), with a passage hole (31b), which crosses the elongate hole (31a) and guides the pin (19) concerned to be displaceable parallelly to the pivot axis of the hinge joint (23) connecting this holder (13) with the connecting member (17) as well as to be rotatable about the axis of the pin, but not to be pivotable in three dimensions with respect to the holder (13), and with a threaded bore (31c) opening into this passage hole (31b) for the retention of a clamping screw (33), wherein the threaded bore (31c) is disposed on that side of the axis (21) of the generally cylindrical carrier portion, which is remote from the axis of the elongate hole.

13. Setting device according to one of claims 1 to 12, characterised thereby, that each of both the holders displays retaining and clamping means (41, 45, 47) of the named kind as well as at least one guide body (49), which is of the named kind, displaceably guides a pin (19) and which is pivotable in three dimensions and firmly clampable in different pivotal settings.

**Revendications**

1. Fixateur pour immobiliser des fragments d'os (1a, 1b), comportant deux supports allongés (13, 15), dans lesquels des tiges (19) destinées à être engagées dans les fragments d'os (1a, 1b) sont agencées de façon à pouvoir être bloquées avec possibilité de réglage de leur propre position longitudinale, et un organe de liaison allongé (17), qui est relié, par chacune de ses extrémités opposées, à l'extrémité avoisinante de l'un des supports (13, 15) par une articulation (23, 25) agencée de façon à pouvoir être immobilisée en diverses positions angulaires, caractérisé en ce que chacune de ces deux articulations (23, 25) comporte une broche d'articulation (63, 73) définissant un axe de pivotement perpendiculaire à la direction longitudinale du support considéré (13, 15) et de l'organe de liaison (17) et en ce qu'au moins un support (15) maintient, à l'aide de moyens de maintien et de serrage (41, 45, 47), au moins un corps de guidage (49) qui guide en coulissement l'une des tiges (19), de façon que ce corps puisse pivoter dans l'espace et être bloqué en diverses positions angulaires.

2. Fixateur selon la revendication 1, caractérisé en ce que les moyens de maintien et de serrage (41, 45, 47) comprennent deux éléments de serrage (41g, 45) et au moins une vis de serrage (47) servant à serrer ceux-ci l'un contre l'autre, en ce que les éléments de serrage (41g, 45) sont munis, sur leurs faces tournées l'une vers l'autre, de creux (41h, 45h) dans lesquels est maintenu le ou l'un au moins des corps de guidage (49) et en ce que le ou chacun des corps de guidage (49) possède une face extérieure en forme de sphère ou de segment sphérique et au moins une entaille (49b) ou fente et est agencé de façon à pouvoir être immobilisé par serrage par les éléments de serrage (41g, 45) et être ainsi comprimé de façon telle qu'à l'état immobilisé par serrage et comprimé, il bloque de son côté la tige (19) qui le traverse.

3. Fixateur selon la revendication 2, caractérisé en ce que les deux éléments de serrage (41g, 45) maintiennent au moins deux corps de guidage (49) du genre indiqué qui guident chacun en translation une tige (19), avec possibilité de pivotement dans l'espace et d'immobilisation par serrage en diverses positions, et en ce qu'il est prévu, pour serrer les deux éléments de serrage (41g, 45) l'un contre l'autre, deux vis de serrage (47) entre lesquelles se trouvent les corps de guidage (49).

4. Fixateur selon l'une des revendications 1 à 3, caractérisé en ce que les extrémités éloignées l'une de l'autre de l'organe de liaison (17) sont agencées de façon à pouvoir pivoter l'une par rapport à l'autre autour d'un axe parallèle à la direction longitudinale de l'organe de liaison (17) et à pouvoir être immobilisées en diverses positions angulaires.

5. Fixateur selon l'une des revendications 1 à 4, caractérisé en ce que l'organe de liaison (17) comporte un manchon (61) à ouverture longitudinale (61g) et une broche (65) capable de pivoter et d'être immobilisée par serrage dans celle-ci en diverses positions angulaires et en ce que l'une (23) des articulations relie l'un (13) des supports au manchon (61) et l'autre articulation (25) relie l'autre support (15) à la broche (65).

6. Fixateur selon la revendication 5, caractérisé en ce que le manchon (61) est muni d'au moins une vis de fixation (67) pour attaquer une surface de serrage (65b, 65c, 65d) de la broche (65) et pour immobiliser cette dernière par serrage, cette surface ne présentant pas de symétrie de révolution autour de l'axe de la broche et étant par exemple plane et/ou formée par une gorge ou étant courbée de façon concave en section transversale, le manchon (61) comportant, pour maintenir la vis de serrage (67), au moins deux trous filetés (61k, 61m) décalés l'un par rapport à l'autre le long du pourtour du manchon, disposés perpendiculairement à son axe longitudinal et débouchant dans son ouverture longitudinale (61g) et/ou la broche (65) comportant au moins deux surfaces de serrage (65b, 65c, 65d) décalées l'une par rapport à l'autre le long de son pourtour, qui ne présentent pas de symétrie de révolution autour de l'axe de la broche et que la vis de serrage (67) peut attaquer au choix.

7. Fixateur selon l'une des revendications 1 à 6, caractérisé en ce que l'organe de liaison (17) comporte un manchon (61) à ouverture longitudinale (61g) et une broche (65) guidée dans celle-ci de façon à pouvoir se déplacer en translation axialement et susceptible d'être immobilisée par serrage en diverses positions de translation, en ce que l'une (23) des articulations relie l'un (13) des supports au manchon (61) et l'autre articulation (25) relie l'autre support (15) à la broche (65), en ce que la broche (65) possède un trou alongé (65e) dont au moins une partie est munie d'un filetage intérieur et en ce qu'une vis de serrage (69) vissé dans ce dernier est présente avec une tête accessible de l'extérieur du manchon (61) pour tirer la broche (65) contre l'articulation (23) reliant le manchon (61) à l'un (13) des supports et par conséquent les deux supports (13, 15) l'un contre l'autre.

8. Fixateur selon la revendication 7, caractérisé en ce que, à une extrémité, le manchon (61) possède un appendice (61d) qui est excentré par rapport à l'axe longitudinal de celui-ci et qui constitue l'articulation (23) reliant le manchon (61) à l'un (13) des supports, en coopération avec une partie de l'un (13) des supports et avec l'une (63) des broches d'articulation, et en ce que la vis de serrage (69) est excentrée par rapport à l'axe de celle (61h) des parties de l'ouverture longitudinale (61g) du manchon qui guide la broche (65) et traverse une partie terminale (61i) de l'ouverture longitudinale (61g) du manchon qui est décalée vers le côté, éloigné de l'appendice (61d), de l'axe de celle (61h) des parties de l'ouverture longitudinale (61g) du manchon qui guide la broche (65), l'appendice (61d) comportant de préférence un évidement (61n) dans lequel la tête de la vis de serrage (69) s'engage de préférence avec jeu axial de telle façon que, par desserrage de la vis de serrage (69), il puisse être produit une force tendant à écarter la broche (65) de l'appendice (61d).

9. Fixateur selon l'une des revendications 1 à 8, caractérisé en ce que l'organe de liaison (17) comporte des moyens de guidage et de serrage (65g, 65h, 71) pour immobiliser par serrage au moins une tige (19) en position longitudinale, avec possibilité de réglage de celle-ci, ces moyens de guidage et de serrage (65g, 65h, 71) guidant la tige (19) en translation, de préférence sans possibilité de pivotement dans l'espace, parallèlement à l'axe de pivotement d'au moins l'une des deux articulations (23, 25).

10. Fixateur selon l'une des revendications 1 à 9, caractérisé en ce que la broche (63, 73) de chaque articulation (23, 25) possède une partie filetée, une partie d'appui et une tête et en ce que l'articulation (23, 25) est susceptible d'être bloquée par vissage de la broche d'articulation correspondante (63, 73).

11. Fixateur selon l'une des revendications 1 à 10, caractérisé en ce que les supports (13, 15) et l'organe de liaison (17) comportent, sur les articulations (23, 25), des surfaces d'appui (31e, 41e, 61e, 65k) reposant deux par deux les unes sur les autres et munies de dentures (65n) dont les dents sont réparties autour de l'axe de pivotement de l'articulation (23, 25) considérée, de façon telle qu'elles puissent engrener l'une avec l'autre en diverses positions angulaires des surfaces d'appui (31e, 41e, 61e, 65k).

12. Fixateur selon l'une des revendications 1 à 11, caractérisé en ce que l'un (13) des supports comprend un bras (31) dont une partie a une forme générale cylindrique de révolution et qui est muni d'un trou longitudinal excentré (31a) et, pour chaque broche (19) tenue par ce bras (13), d'un trou de passage (31b) croisant le trou longitudinal (31a) et guidant la tige (19) considérée en translation parallèlement à l'axe de pivotement de l'articulation (23) reliant ce bras (13) à l'organe de liaison (17) et en rotation autour de l'axe de la tige, mais sans possibilité de pivotement dans l'espace par rapport au bras (13), ainsi que d'un trou fileté (31c) débouchant dans le trou de passage (31b) et servant à maintenir une vis de serrage (33), le trou fileté (31c) étant situé sur celui des côtés de l'axe (21) de la partie de forme générale cylindrique du bras qui est éloigné de l'axe du trou longitudinal.

13. Fixateur selon l'une des revendications 1 à 12, caractérisé en ce que chacun des deux bras possède des moyens de maintien et de serrage (41, 45, 47) du genre indiqué ainsi qu'au moins un corps de guidage (49) du genre indiqué guidant une tige (19) en translation et susceptible de pivoter dans l'espace et d'être immobilisé par serrage en diverses positions angulaires.

Fig.1

Fig.2

Fig. 3

Fig. 4

Fig.5

# Fig. 6

69  61i
61
61d
61a
61h

# Fig. 8

65b
65c
+15
+10
+5
0
-5
-10
-15
65a
65
65f

# Fig. 7

67  61k
65c  65d
61b  65a
61m  61
61h
VIII
65b  65e

65  65f
65h  65g
65i
65m
65k
65n

# Fig. 9